# EUROPÄISCHE PATENTSCHRIFT

(11) **EP 1 221 438 B1**
(45) Veröffentlichungstag und Bekanntmachung des Hinweises auf die Patenterteilung: **14.03.2007**
(21) Anmeldenummer: 01129690.2
(22) Anmeldetag: 13.12.2001
(51) Int. Cl.: C07D 211/90, A61K 31/4422, A61P 9/10, A61P 9/12

(54) **Salze von Amlodipin-Mesylat**
Amlodipine mesylate salts
Sels d'amlodipine mésylate

(30) Priorität: 09.01.2001 CH 222001
(43) Veröffentlichungstag der Anmeldung: 10.07.2002
(73) Patentinhaber: Siegfried Generics International AG, 4800 Zofingen (CH)
(72) Erfinder: Hoss, Ralf, Dr., 4800 Zofingen (CH); Oelting, Michael, 4800 Zofingen (CH); Müller, Beat W., Dr., 4106 Therwil (CH)
(74) Vertreter: Braun, André jr.

(56) Entgegenhaltungen:
- EP-A- 0 244 944
- WO-A-99/52873
- DATABASE WPI Section Ch, Week 200064 Derwent Publications Ltd., London, GB; Class B03, AN 2000-656957 XP002165755 & CN 1 263 093 A (KUNMING PHARM CO LTD), 16. August 2000 (2000-08-16)

## Beschreibung

Die vorliegende Erfindung betrifft ein ausgewähltes Salz von Amlodipin-Mesylat. Insbesondere weist diese Modifikation verbesserte Eigenschaften bezüglich ihrer Einsetzbarkeit als pharmazeutisch wirksame Substanz auf, wie dies im weiteren beschrieben ist.

Amlodipin-Mesylat entspricht der Formel (I) 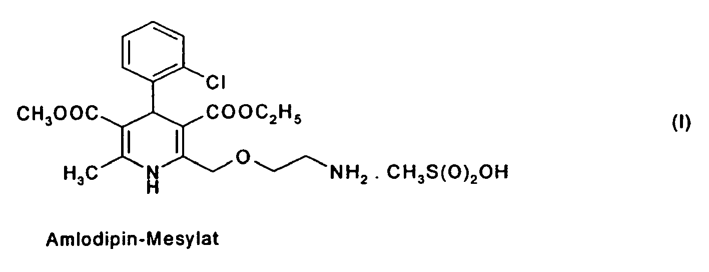 und ist das Methansulfonsäuresalz von Amlodipin. Amlodipin wird chemisch als 2-[2-(Aminoethoxy)methyl]-4-(2-chlorphenyl)-3-ethoxycarbonyl-5-methoxycarbonyl-6-methyl-1,4-dihydropyridin bezeichnet und ist an sich bekannt. Amlodipin wird als pharmazeutisch wirksamer Calcium-Antagonist mit ausgezeichneter anti-ischaemischer und anti-hypertensiver Wirkung eingesetzt.

EP 0 244 944 beschreibt ohne nähere präparative und analytische Angaben verschiedene Salze von Amlodipin, wie beispielsweise das Benzolsulfonatsalz (Besylat), das Toluolsulfonatsalz (Tosylat), sowie z.B. auch Salze der Amlodipinbase mit Maleinsäure, Fumarsäure, Milchsäure oder Zitronensäure. Auch Amlodipin-Mesylat ist beschrieben, jedoch wird darauf hingewiesen, dass Amlodipin-Mesylat ein hygroskopisches Salz darstellt, welches für die Tablettierung ungeeignet ist.

CN 1263093 beschreibt ein in Wasser recht stabiles, Amlodipin-Mesylat Monohydrat mit einem Schmelzpunkt im Bereich von 139°C bis 143°C, nennt jedoch keine wasserfreie Form. Das beschriebene Amlodipin-Mesylat Monohydrat ist nicht hygroskopisch bzw. in feuchter Luft stabil.

Es wurde nun überraschenderweise gefunden, dass Amlodipin-Mesylat in einer kristallinen Form (Modifikation) hergestellt werden kann, wobei es für die Verarbeitung zu pharmazeutisch wirksamen Verabreichungsformen geeignet ist. Insbesondere weist die Modifikation ihre eigenen speziellen Eigenschaften und Vorzüge auf und ist der bis anhin bekannten hygroskopischen Form deutlich überlegen.

Die vorliegende Erfindung ist in den Patentansprüchen definiert. Insbesondere betrifft die vorliegende Erfindung Salz von Amlodipin mit Methansulfonsäure (Amlodipin-Mesylat) in einer Modifikation, welche dadurch gekennzeichnet ist, dass
die Modifikation (im weiteren als Modifikation A bezeichnet) einen Schmelzpunkt von 184±4°C hat, und im Pulver-Röntgendiagramm charakteristische Peaks bei 6.6 (sehr stark), 10.2 (schwach), 11.0 (sehr stark), 14.7 (schwach) und 15.9 (mittel) °2 Theta aufweist.

Vorzugsweise ist die Modifikation A im wesentlichen wasserfrei und hat einen Wassergehalt von ≤0.2 Gew.-%.

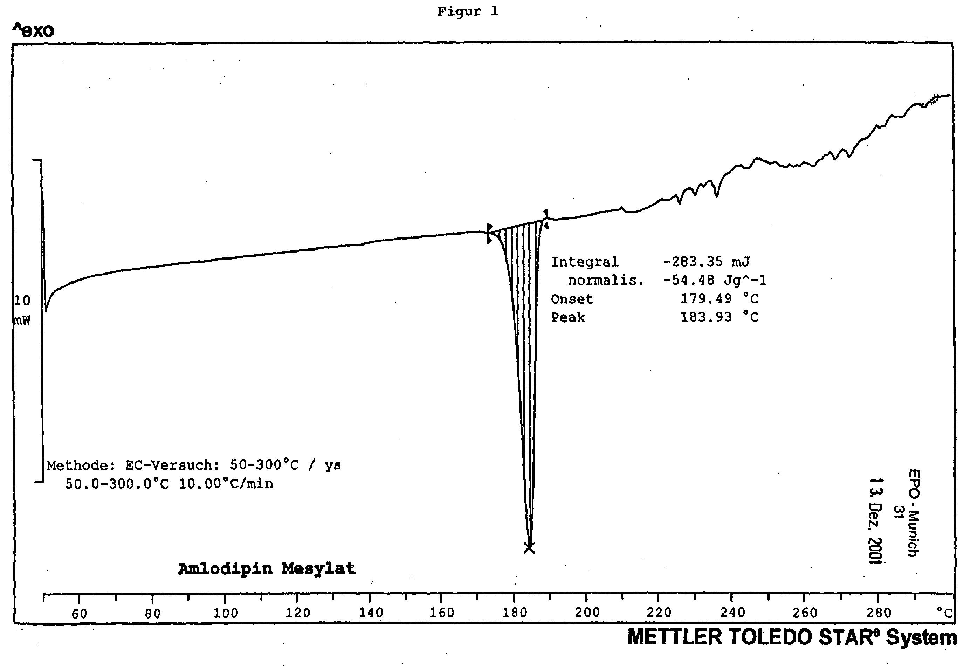

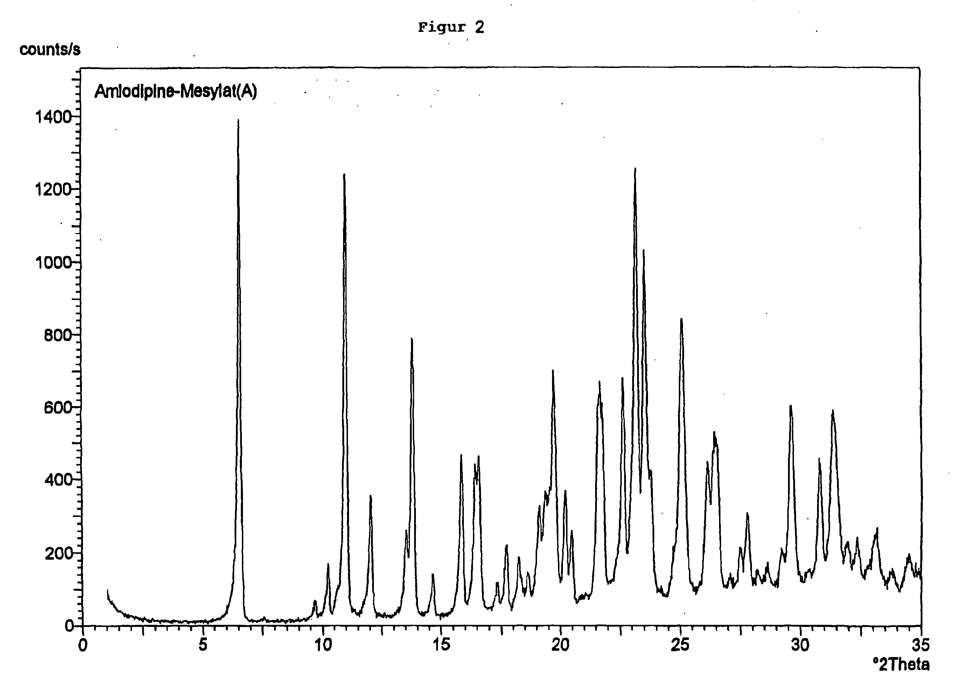

Die angegebenen Schmelzpunkte wurden mittels Differential Scanning Calorimetry (DSC) gemessen. Die Messresultate für die Schmelzpunkte sind für die Modifikation A aus Figur 1 ersichtlich.

Die Untersuchungsergebnisse mittels Röntgenstrahlen sind für die Modifikation A im Röntgen-Pulverdiffraktogramm der Figur 3 dargestellt.

Die vorliegende Erfindung betrifft auch Herstellungsverfahren für die Modifikation von Amlodipin-Mesylat sowie pharmazeutisch wirksame Zusammensetzungen, welche die Modifikation enthalten. Die vorliegende Erfindung betrifft im weiteren die Verwendung der beanspruchten Modifikation von Amlodipin-Mesylat für die Behandlung von ischaemischen Herzerkrankungen, insbesondere für die Behandlung von angina pectoris oder Bluthochdruck im menschlichen Körper. Die Erfindung betrifft auch die Verwendung der beanspruchten Modifikation von Amlodipin-Mesylat für die Herstellung von Heilmitteln, insbesondere in Form von Tabletten, Pulvern oder Granulaten, gegebenenfalls abgefüllt in Kapseln, sowie wässerige Lösungen für die parenterale Verabreichung, insbesondere für die Behandlung von ischaemischen Herzerkrankungen, wie angina pectoris oder Bluthochdruck.

Wie bereits erwähnt, erhält man Amlodipin-Mesylat mit der bis anhin bekannten Herstellungsmethode als hygroskopisches Salz, welches für die Tablettierung ungeeignet ist. Insbesondere kann es schlecht verarbeitet werden und bereitet bei der Dosierung Schwierigkeiten, da ein sich unkontrolliert verändernder Wassergehalt eine genaue Dosierung praktisch verunmöglicht. Gegenüber der bis anhin bekannten hygroskopischen Form hat die erfindungsgemässe Modifikation A deutliche Vorteile.

Die wasserfreie Kristallform A ist in üblichen festen galenischen Formulierungen stabil, so dass selbst nach achtmonatiger Lagerung bei 40°C und 75% relativer Luftfeuchtigkeit keine Wasseraufnahme mittels Röntgenpulverdiffraktion nachgewiesen werden kann. Solche galenische Formulierungen, welche die Aktivsubstanz vor Wasseraufnahme schützen, sind an sich bekannt.

Die Kristallform erlaubt eine definierte Dosierung des pharmazeutisch aktiven Wirkstoffs und zeigt im Gegensatz zur bisher beschriebenen Form keine nachteiligen Auswirkungen auf Prozess relevante Vorgänge wie Sieben, Mischen oder Tablettieren.

Das Amlodipin-Mesylat weist gegenüber dem Amlodipin-Besylat eine um den Faktor fünf höhere Wasserlöslichkeit auf (25 mg/ml versus 4.6 mg/ml).

Für die Herstellung des Amlodipin-Mesylats geht man vorzugsweise so vor, dass man in inerter Atmosphäre die Amlodipinbase und Methansulfonsäure in praktisch stöchiometrischen Mengen unter Verwendung eines Lösungsmittels zur Reaktion bringt. Man löst Amlodipin und Methansulfonsäure, vorzugsweise separat, in einem geeigneten Lösungsmittel, beispielsweise in 2-Propanol bei einer Temperatur von ca. 40°C, bringt anschliessend beide Komponenten bei dieser Temperatur zusammen und lässt abkühlen. Dabei kristallisiert das Mesylat aus. Man kann auch Methansulfonsäure unverdünnt zum gelösten Amlodipin hinzu fügen oder umgekehrt Amlodipin unverdünnt zur gelösten Methansulfonsäure hinzu fügen und das Mesylat auskristallisieren lassen.

Die wasserfreie Modifikation A wird beispielsweise erhalten, wenn man Amlodipinbase unter Stickstoffatmosphäre in einem geeigneten wasserfreien Lösungsmittel, beispielsweise in wasserfreiem 2-Propanol, löst und, gegebenenfalls nach Filtration, Methansulfonsäure, gegebenenfalls in gelöster Form, beispielsweise gelöst in wasserfreiem 2-Propanol, hinzu tropft.

Die erfindungsgemässe Modifikation von Amlodipin-Mesylat kann in an sich bekannter Weise für die Herstellung von Heilmitteln, insbesondere für die Herstellung von Tabletten, Pulvern oder Granulaten, oder von wässerigen Lösungen, verwendet werden. Auch können Granulate in an sich bekannter Weise in Hart- oder Weichgelatinekapseln abgefüllt werden. Die folgenden Beispiele illustrieren die Erfindung.

### Beispiel 1 (Herstellung der Modifikation A)

163.6 Gramm (0.40 Mol) Amlodipinbase werden in 940 Gramm wasserfreiem 2-Propanol bei ca. 35-40°C unter Stickstoffatmosphäre gelöst. Die gelbliche, leicht trübe Lösung wird filtriert und auf 25-30°C abgekühlt. Anschliessend tropft man während 30-50 Minuten 39.3 Gramm (0.41 Mol) Methansulfonsäure hinzu und rührt für weitere 1-2 Stunden bei 25-30°C Innentemperatur. Nach Abkühlung der Lösung auf 10-17°C Innentemperatur wird für mindestens 2 Stunden weiter gerührt. Die Kristalle werden anschliessend abfiltriert, mit 2-Propanol gewaschen und im Vakuumtrockner bei 40°C getrocknet. Die auf die Base bezogene Ausbeute beträgt 94%. Schmelzpunkt: 185.3°C; Wassergehalt (bestimmt nach Karl-Fischer): 0.08 Gew.-%. Die Untersuchungsergebnisse mittels Röntgenstrahlen zeigen, dass die Modifikation A gemäss dem Röntgen-Pulverdiffraktogramm der Figur 3 erhalten wurde.

### Beispiel 2 (Verarbeitung der Modifikation A zu einer Tablette)

Es wurden Tabletten hergestellt enthaltend jeweils 6.175 mg bzw. 12.35 mg wasserfreies Amlodipin-Mesylat (Modifikation A) entsprechend 5 mg bzw. 10 mg wasserfreier Amlodipin-Base (Modifikation A). Dabei wurden als galenische Hilfsstoffe verwendet: mikrokristalline Cellulose, wasserfreies Kalziumhydrogenphosphat, Natriumcarboxymethylstärke und Magnesiumstearat. Die überlagerten Pulverröntgendiffraktorgramme zeigten, dass selbst nach achtmonatiger Lagerung bei 40°C und 75% relativer Luftfeuchtigkeit keine Wasseraufnahme statt gefunden hatte.

## Patentansprüche

1. Salz von Amlodipin mit Methansulfonsäure (Amlodipin-Mesylat) in einer ausgewählten Modifikation, **dadurch gekennzeichnet, dass** diese Modifikation (Modifikation A) einen Schmelzpunkt von 184±4°C hat, und im Pulver-Röntgendiagramm charakteristische Peaks bei 6.6 (sehr stark), 10.2 (schwach), 11.0 (sehr stark), 14.7 (schwach) und 15.9 (mittel) °2 Theta aufweist.

2. Amlodipin-Mesylat nach Anspruch 1 als wasserfreie Modifikation A mit einem Wassergehalt von ≤0.2 Gew.-%.

3. Verfahren zur Herstellung der wasserfreien Modifikation A nach Anspruch 1 oder 2, **dadurch gekennzeichnet, dass** man Amlodipinbase unter Stickstoffatmosphäre in einem geeigneten wasserfreien Lösungsmittel, vorzugsweise in wasserfreiem 2-Propanol, löst und, gegebenenfalls nach Filtration, Methansulfonsäure, gegebenenfalls in gelöster Form, hinzu tropft.

4. Verwendung von Amlodipin-Mesylat gemäss einem der Ansprüche 1 oder 2 für die Herstellung von Heilmitteln, insbesondere für die Herstellung von Tabletten, Pulvern oder Granulaten, oder von wässerigen Lösungen.

5. Pharmazeutisch wirksame Zusammensetzungen für die Behandlung von ischaemischen Herzerkrankungen, insbesondere für die Behandlung von angina pectoris oder Bluthochdruck im menschlichen Körper, **dadurch gekennzeichnet, dass** diese mindestens ein Amlodipin-Mesylat gemäss einem der Ansprüche 1 oder 2 enthalten.

6. Tabletten, Pulver oder Granulate, gegebenenfalls abgefüllt in Kapseln, sowie wässerige Lösungen für die parenterale Verabreichung, nach Anspruch 5.

## Claims

1. Salt of amlodipine with methanesulfonic acid (amlodipine mesylate) in a selected modification, **characterized in that** this modification (modification A) has a melting point of 184 ± 4°C and characteristic peaks in the X-ray powder diagram at 6.6 (very strong), 10.2 (weak), 11.0 (very strong), 14.7 (weak) and 15.9 (moderate) °2 theta.

2. Amlodipine mesylate according to Claim 1 as an anhydrous modification A with a water content of ≤0.2% by weight.

3. Process for the preparation of the anhydrous modification A according to Claim 1 or 2, **characterized in that** amlodipine base is dissolved under a nitrogen atmosphere in a suitable anhydrous solvent, preferably in anhydrous 2-propanol, and, optionally after filtration, methanesulfonic acid, optionally in dissolved form, is added dropwise.

4. Use of amlodipine mesylate according to Claim 1 or 2 for the preparation of medicaments, especially for the preparation of tablets, powders or granules, or aqueous solutions.

5. Pharmaceutically effective compositions for the treatment of ischaemic heart diseases, especially for the treatment of angina pectoris or high blood pressure in the human body, **characterized in that** they contain at least one amlodipine mesylate according to Claim 1 or 2.

6. Tablets, powders or granules, optionally filled into capsules, and aqueous solutions for parenteral administration, according to Claim 5.

## Revendications

1. Sel d'amlodipine avec de l'acide méthanesulfonique (amlodipine mésylate) en une modification choisie, **caractérisé en ce que** cette modification (modification A) a un point de fusion de 184 ± 4°C et comprend dans le diagramme de rayons X de poudre des pics caractéristiques à 6.6 (très fort), 10.2 (faible), 11.0 (très fort), 14.7 (faible) et 15.9 (moyen) °2 théta.

2. Amlodipine mésylate selon la revendication 1 sous forme de modification A anhydre avec une teneur en eau ≤ 0.2 % en poids.

3. Procédé pour la fabrication de la modification A anhydre selon les revendications 1 ou 2, **caractérisé en ce que** l'on dissout la base d'amlodipine sous atmosphère d'azote dans un solvant anhydre approprié, de préférence dans du 2-propanol anhydre, et optionnellement après filtration, on y ajoute goutte à goutte de l'acide métanesulfonique, optionnellement sous forme dissoute.

4. Utilisation d'amlodipine mésylate selon l'une quelconque des revendications 1 ou 2 pour la fabrication de médicaments, en particulier pour la fabrication de comprimés, poudres ou granulés ou de solutions aqueuses.

5. Compositions pharmaceutiquement efficaces pour le traitement de maladies cardiaques ischémiques, en particulier pour le traitement d'angina pectoris ou de haute pression sanguine dans le corps humain, **caractérisées en ce que** celles-ci comprennent au moins un amlodipine mésylate selon l'une quelconque des revendications 1 ou 2.

6. Comprimés, poudres ou granulés, optionnellement remplis dans des capsules, ainsi que des solutions aqueuses pour l'administration parentérale selon la revendication 5.
